# EUROPÄISCHE PATENTSCHRIFT

(11) **EP 1 814 844 B1**
(45) Veröffentlichungstag und Bekanntmachung des Hinweises auf die Patenterteilung: **16.09.2009**
(21) Anmeldenummer: 05803766.4
(22) Anmeldetag: 18.11.2005
(51) Int. Cl.: C07C 231/02

(54) **VERFAHREN ZUR KONTINUIERLICHEN HERSTELLUNG VON ALKYLAMINOACRYLAMIDEN**
METHOD FOR THE CONTINUOUS PRODUCTION OF ALKYL AMINO ACRYL AMIDES
PROCEDE DE PRODUCTION CONTINUE D'ALKYLAMINOACRYLAMIDES

(30) Priorität: 23.11.2004 DE 102004056629
(43) Veröffentlichungstag der Anmeldung: 08.08.2007
(73) Patentinhaber: Evonik Röhm GmbH, 64293 Darmstadt (DE)
(72) Erfinder: SCHLEEP, Volker, 64683 Einhausen (DE); MERTZ, Thomas, 64625 Bensheim (DE)
(86) Internationale Anmeldenummer: PCT/EP2005/012362
(87) Internationale Veröffentlichungsnummer: WO 2006/056366

(56) Entgegenhaltungen:
- WO-A-20/04103952
- DE-A1- 4 027 843
- DE-A1- 10 323 699

## Beschreibung

### Gebiet der Erfindung

Die Erfindung betrifft ein kontinuierlich betriebenes Verfahren zur Herstellung von Alkylaminoacrylamiden (C) durch kontinuierliche Aminolyse von beispielsweise Butylacrylat (A mit R₁ = C₄) mit Aminen (B) unter Freisetzung von Butanol (D mit R₁= C₄) gemäß folgender Reaktionsgleichung: 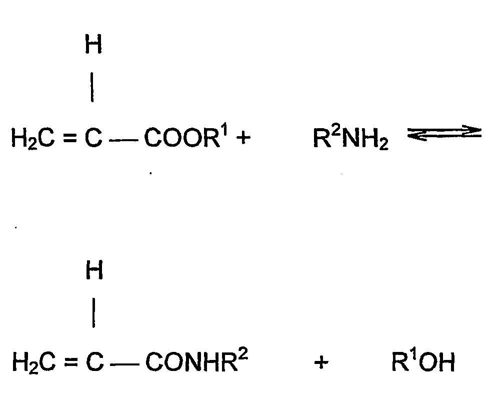 wobei gilt:
- R¹ =: linearer oder verzweigter Alkylrest mit 3 bis 10 Kohlenstoffatomen
- R²: steht für einen linearen, verzweigten oder cyclischen Alkylrest, einen Arylrest, der auch mit ein oder mehreren Alkylgruppen subsituiert sein kann, der lineare, cyclische oder verzweigte Alkylrest kann eine Länge von 1-12 Kohlenstoffatomen aufweisen, wie beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl bedeuten und kann gegebenenfalls durch
- NR³ R⁴ oder
- OR⁵
   ein- oder mehrfach substituiert sein, dabei kann entweder R³ oder R⁴ die Bedeutung von Wasserstoff annehmen und ferner gilt:
- R³, R⁴ oder R⁵ können entweder gleich oder verschieden sein und eine Alkylgruppe mit 1 - 12 Kohlenstoffatomen darstellen, wie beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl oder Wasserstoff.
- R² kann ferner auch

   [(R⁶-0)ₙ]-R⁷

   bedeuten, wobei gilt:
- R⁶ kann eine C₁-C₄-Alkylgruppe sein, die auch verzweigt sein kann, wie beispielsweise Methyl, Ethyl, Propyl, Isopropyl, Butyl, Isobutyl oder tert. Butyl.
   Alkylamido(meth)acrylate
   m:1- 4
   R⁷ kann die Methylgruppe oder die Ethylgruppe sein.

Als Amine kommen beispielsweise folgende Verbindungen in Frage:

Dimethylaminoethylamin, Diethylaminoethylamin, Dipropylaminoethylamin, Diisopropylaminoethylamin, Dibutylaminoethylamin, Disobutylaminoehtylamin, Dimethylaminopropylamin, Diethylaminopropylamin, Dipropylaminopropylamin, Düsopropylaminopropylamin, Dibutylaminopropylamin, Disobutylaminopropylamin, Dimethylaminobutylamin, Diethylaminobutylamin, Dipropylaminobutylamin, Diisopropylaminobutylamin, Dibutylaminobutylamin, Disobutylaminobutylamin, Methylamin,-Cyclohexylamin, Dimethylaminohexylamin, Diethylaminohexylamin.

Besonders bevorzugt ist neben Dimethylaminopropylamin, Dimethylaminoethylamin, Dimethylaminobutylamin, Dimethylaminopentylamin und Dimethylaminohexylamin.

### Stand der Technik

In der Literatur sind viele diskontinuierlich durchgeführte Umesterungsverfahren (Batch-Umesterungsverfahren) in Verbindung mit unterschiedlichen Katalysatoren beschrieben.

Die Suche nach wirtschaftlicheren Verfahren führte zu der Auffindung von kontinuierlichen Umesterungsverfahren, bei denen die Edukte kontinuierlich zugeführt und die Produkte kontinuierlich abgeführt werden. Die kontinuierlichen Umesterungsverfahren haben gegenüber den diskontinuierlichen Umesterungsverfahren folgende Vorteile: Der Prozess ist leichter automatisierbar und kann mit reduziertem Personalbedarf betrieben werden, die Produktqualität ist besser reproduzierbar und weniger schwankend, die Anlagenkapazität erhöht sich aufgrund des Wegfalls des sequenziellen Abarbeitens der einzelnen Herstellungsschritte (Befüllen, Reaktion, Niedrigsiederabtrennung, Produktabtrennung, Entleeren). Der Prozess besitzt eine höhere Raum-Zeit-Ausbeute als ein Batch-Prozess.

Kontinuierliche Umesterungsverfahren sind bekannt.

EP 0 960 877 (Elf Atochem S.A.) beschreibt ein kontinuierliches Verfahren zur Herstellung von Methacrylatestern von Dialkylaminoalkoholen. Man setzt Dialkylaminoalkohole mit im allgemeinen Methyl(meth)acrylat um und erhält das Dialkylaminoalkyl(meth)acrylat nach folgendem Verfahren:

Das Gemisch der Ausgangsstoffe (Methyl(meth)acrylat und Dialkylaminoalkohol) wird zusammen mit einem Tetraalkyltitanat als Katalysator (beispielsweise Tetrabutyl-, Tetraethyl- oder Tetra(2-Ethylhexyl)titanat) und mindestens einem Polymerisationsinhibitor (beispielsweise Phenothiazin, tert.-Butylcatechol, Hydrochinonmonomethylether oder Hydrochinon) kontinuierlich einem Rührreaktor zugeführt, wo bei einer Temperatur von 90 -120 °C die Umsetzung zum Dialkylamino(meth)acrylat bei gleichzeitigem kontinuierlichem Abzug des azeotropen Methyl(meth)acrylat/Methanol-Gemisches erfolgt. Das rohe Reaktionsgemisch (Rohester) wird einer ersten Destillationskolonne zugeführt, wobei man bei vermindertem Druck am Kopf der Destillationskolonne einen im wesentlichen katalysatorfreien Strom abzieht und im Sumpf der Destillationskolonne der Katalysator sowie wenig Dialkylaminoalkyl(meth)acrylat abgezogen werden. Der Kopfstrom der ersten Destillationskolonne wird dann einer zweiten Destillationskolonne zugeführt, bei der man unter vermindertem Druck am Kopf einen Strom von niedrigsiedenden Produkten mit wenig Dialkylaminoalkyl(meth)acrylat und im Sumpf einen Strom bestehend aus hauptsächlich Dialkylaminoalkyl(meth)acrylat sowie Polymerisationsinhibitor(en) abzieht, der einer dritten Destillationskolonne zugeführt wird. In der dritten Destillationskolonne wird unter vermindertem Druck eine Rektifikation durchgeführt, in der man am Kopf den gewünschten reinen Dialkylaminoalkyl(meth)acrylatester und im Sumpf im wesentlichen den Polymerisationsinhibitor oder die Polymerisationsinhibitoren abzieht. Der Sumpfstrom der ersten Destillationskolonne wird nach weiterer Aufreinigung mit Hilfe eines Filmverdampfers genauso in den Reaktor zurückgeführt wie der Kopfstrom aus der zweiten Destillationskolonne.

Dieses Verfahren verzichtet auf eine Entwässerung der Alkohole vor dem Einsatz, was zu einer verstärkten Desaktivierung des eingesetzten Tetraalkyltitanats infolge Hydrolyse bis hin zur Bildung unerwünschter Feststoffniederschläge führen kann. Weiterhin verfügt das Verfahren über den Nachteil, dass in der ersten Destillationskolonne der Katalysator im Sumpf bei relativ hohen Temperaturen thermisch beansprucht wird. Dies kann leicht zur Zersetzung des Katalysators führen.

Bei diesem Verfahren werden sowohl die nicht umgesetzten Edukte wie auch das Produkt insgesamt zweimal über Kopf rektifiziert. Dies erfordert sehr hohe Energiekosten und insgesamt 4 Rektifikationskolonnen, die zum Teil sehr groß dimensioniert sein müssen. Der Prozeß ist daher mit sehr hohen Investitions- und Betriebskosten belastet.

EP 0 968 995 (Mitsubishi Gas Chemical Comp.) beschreibt ein kontinuierliches Verfahren zur Herstellung von Alkyl(meth)acrylsäureestern unter Verwendung einer Reaktionskolonne. Die Umesterungsreaktion erfolgt dabei direkt in einer Destillationskolonne (d.h. Reaktor und Destillationskolonne zum Abzug des Methyt(meth)acrylat/Methanol-Azeotrops bilden einen Apparat), der die Ausgangsstoffe (Methyl(meth)acrylat und Alkohol) kontinuierlich zugeführt werden. Der notwendige Katalysator, hier ebenfalls bevorzugt eine Titanverbindung, befindet sich in der Destillationskolonne. Im Fall eines homogenen Katalysators wird der Katalysator in die Destillationskolonne kontinuierlich eindosiert. Die Verwendung von homogenen Katalysatoren in einer Destillationskolonne führt jedoch aufgrund eines Spüleffektes durch den flüssigen Rücklauf in der Destillationskolonne zu einem erhöhten Katalysatorbedarf sowie bei Auftreten eines Katalysatorfeststoffniederschlags zur Verschmutzung der Kolonneneinbauten. Im Fall eines heterogenen Katalysators befindet sich der Katalysator in der Reaktionskolonne. Die Positionierung des Katalysators in der Destillationskolonne ist allerdings nachteilig, weil in der Destillationskolonne dann ein erhöhter Druckverlust auftritt und zusätzlich für die regelmäßige Reinigung der Destillationskolonne ein sehr hoher Aufwand betrieben werden muss. Weiterhin können heterogene Katalysatoren beispielsweise infolge unerwünschter Polymerisation desaktivieren.

DE 4 027 843 (Röhm GmbH) beschreibt ein kontinuierliches Verfahren zur Herstellung N-substituierter (Meth)acrylsäureamiden durch Aminolyse von Alkylestern der (Meth)acrylsäure mit aliphatischen und aromatischen Aminen. Die Reaktionstemperatur liegt bei > 150°, der Druck beträgt ca. 160 bar. Es wird ohne Katalysator gearbeitet.

### Aufgabe

Aufgabe der vorliegenden Erfindung ist es, ein kontinuierliches Verfahren zur Aminolyse von Acrylsäureestern zur Verfügung zu stellen, das die Nachteile der beiden vorgenannt beschriebenen Verfahren vermeidet. Weiterhin soll durch das neue Verfahren ein Produkt zur Verfügung gestellt werden, das in der Qualität besser ist als die bisher auf dem Markt befindlichen. Unter einer besseren Qualität wird ein geringerer Vernetzergehalt oder ein geringerer Gehalt an Additionsprodukten der Amine an die Doppelbindung des Ausgangsesters oder an die Doppelbindung des Produktamids verstanden.

Ferner sollen mit dem neuen Verfahren Aminoacrylate mit möglichst wenig Aufwand und energetisch günstiger (d.h. kostengünstiger) hergestellt werden. Der Personalaufwand zur Bedienung der Anlage soll verringert werden.

Gelöst werden diese sowie weitere im einzelnen nicht näher ausgeführte, jedoch aus der einleitenden Erörterung des Standes der Technik ohne weiteres erschließbare oder ableitbare Aufgaben durch ein Verfahren mit den Merkmalen des Patentanspruchs 1. Vorteilhafte Abwandlungen des erfindungsgemäßen Verfahrens werden in den auf Anspruch 1 rückbezogenen Ansprüchen unter Schutz gestellt.

### Verfahrensbeschreibung

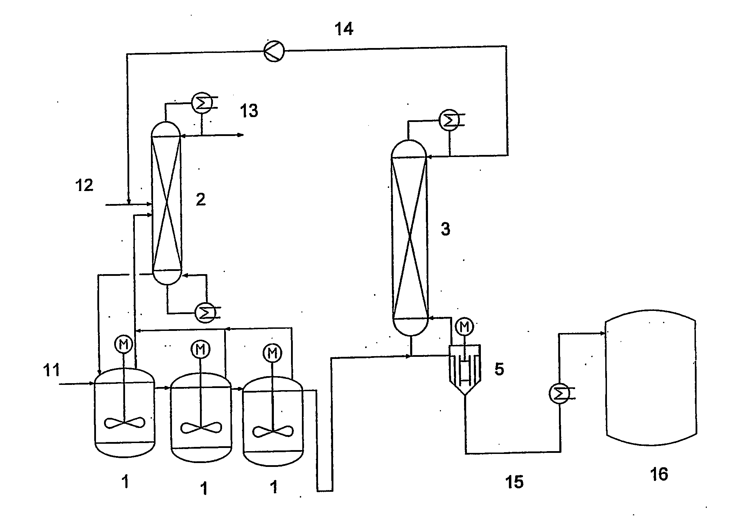

Das Verfahren ist schematisch in Figur 1 dargestellt.

Erläuterungen der Bezugszeichen Figur 1:
1. Reaktionsapparat
2. Niedersieder-Austrags-Destillationskolonne
3. Niedrigsieder-Destillationskolonne
5. Filmverdampfer
11. Acrylat-Feed und Katalysator-Feed
12. Amin-Feed
13. Niedersieder-Austrag
14. Niedrigsieder-Kreislaufstrom
15. Rohprodukt
16. Vorlagebehälter für weitere Destillationsschritte

Der Acrylatfeed Edukt (11) wird kontinuierlich einem geeigneten Reaktionsapparat (1) zugeführt, wobei sowohl ein einzelner Reaktionsbehälter als auch eine Kaskade von mehreren hintereinander geschaltenen Reaktionsbehältern eingesetzt werden kann. Eine solche Kaskade kann beispielsweise aus 2, 3, 4, 5, 6 oder gegebenenfalls mehreren einzelnen Reaktionsbehältern bestehen. In einer bevorzugten Ausführungsform wird eine Kaskade aus 3 hintereinander angeordneten kontinuierlich betriebenen Rührkesseln verwendet.

Der Acrylatfeed Edukt (11) kann auf verschiedene Weise erfolgen. Es ist zum Beispiel möglich, den Eduktstrom (11) nur dem ersten Reaktionsbehälter der Kaskade zuzuführen oder aber den Eduktstrom (11) in Teilströme aufzuteilen und diese Teilströme allen oder nur einigen der hintereinander geschalteten Reaktionsbehältern der Kaskade zuzuführen. Genau so ist es möglich, die Zuführung des Eduktstroms (11) über den Apparat (2) und/oder die Reaktionsapparate (1) vorzunehmen. Es kann vorteilhaft sein, den Eduktstrom (11) nur in den Apparat (2) zuzuführen oder in einer weiteren Ausführungsform den Eduktstrom (11) in Teilströme aufzuteilen, welche dann sowohl dem Apparat (2) als auch dem ersten oder gegebenenfalls mehreren Reaktionsbehältern der Kaskade zugeführt werden.

Es ist sinnvoll, dass alle Reaktionsbehälter einen Brüdenabzug zur Destillationskolonne (2) zur Entfernung des bei der Reaktion frei werdenden Alkohols besitzen.

Die Strömungsführung zu den Reaktoren und aus diesen heraus muss nicht zwangsläufig wie im Fließbild dargestellt erfolgen. Es hat sich in besonderen Ausführungsformen als vorteilhaft erwiesen, den Austrag eines Kessels der Kaskade in den jeweils nachfolgenden Kessel der Kaskade von unten einzuführen.

Das Amin (12) wird kontinuierlich der Destillationskolonne (2) zur Entwässerung zugeführt.

Das als Katalysator benötigte Tetraalkoxititanat (der Tetraalkoxititanat-Gehalt in Bezug auf eingesetzten Acrylester A beträgt bevorzugt 0,2 - 4 Gew.-%) wird wie der/die Polymerisationsinhibitor/en ebenfalls bevorzugt kontinuierlich in den Reaktionsapparat (1) zudosiert. Als Aminolysekatalysatoren können aber auch alle aus dem Stand der Technik bekannten Umesterungskatalysatoren eingesetzt werden. Als Katalysatoren kommen beispielsweise Zirkonacetylacetonat und weitere 1.3-Diketonate des Zirkons in Frage, ferner können Mischungen aus Alkalimetallcyanaten oder Alkalimetallthiocyanaten und Alkalimetallhalogeniden eingesetzt werden, ferner Zinnverbindungen, beispielsweise Dioctylzinnoxid, Erdalkalimetalloxide oder Erdalkalimetallhydroxide, wie beispielsweise CaO, Ca(OH)₂, MgO, Mg(OH)₂ oder Gemische aus den vorstehend genannten Verbindungen, ferner Alkalimetallhydroxide, Alkalimetallalkoxide und Lithiumchlorid und Lithiumhydroxid, es können auch Gemische aus den vorstehend genannten Verbindungen mit den vorstehend genannten Erdalkaliverbindungen und den Li-Salzen eingesetzt werden, Dialkylzinnoxide, wie beispielsweise Dioctylzinnoxid, Alkalimetallcarbonate, Alkalimetallcarbonate zusammen mit quartären Ammoniumsalzen, wie beispielsweise Tetrabutylammoniumhydroxid oder Hexadecyltrimethylammoniumbromid, ferner Mischkatalysatoren aus Diorganylzinnoxid und Organylzinnhalogenid, saure lonenaustauscher, Phosphormolybdän-Heteropolysäuren, Titanalkoholate, wie beispielsweise Isopropyltitanat, Chelatverbindungen der Metalle Titan, Zirkonium, Eisen oder Zink mit 1.3-Di-Carbonylverbindungen, Bleiverbindungen, wie beispielsweise Bleioxide, Bleihydroxide, Bleialkoxide, Bleicarbonate oder Bleisalze von Carbonsäuren. Besonders bevorzugt ist ein Katalysatorgemisch aus Dialkylzinnoxid und Alkyltitanat, beispielsweise Dioctylzinnoxid und Isopropyltitanat im Verhältnis ca. 2,5 : 1 (Gew.-%/Gew.-%).

Der Katalysator oder das Katalysatorgemisch wird in Mengen von 0,1 -10 Gew.-%, bevorzugt 0,2 - 7 Gew.-%, jeweils bezogen auf das eingesetzte Acrylat, eingesetzt.

Geeignete Alkylacrylate sind alle Acrylate mit einem linearen oder verzweigten Alkylrest mit 3 bis 10, bevorzugt 3 bis 6 und besonders bevorzugt 3 oder 4 Kohlenstoffatomen. Typische Beispiele hierfür sind Propylacrylat, Isopropylacrylat, n-Butylacrylat, Isobutylacrylat, 3-Methylbutylacrylat, Amylacrylat, Neopentylacrylat, Hexylacrylat, Cyclohexylacrylat, Heptylacrylat, n-Octylacrylat, Ethylhexylacrylat oder Decylacrylat.

Als Amine können alle Verbindungen R²NH₂ verwendet werden, deren Rest R² aus 1-12, bevorzugt 2-8 oder besonders bevorzugt aus 2-4 Kohlenstoffatomen besteht. Beispiele für typische Strukturen und konkrete Verbindungen sind bereits zu Beginn dieser Anmeldung aufgeführt.

Es ist für den Fachmann des Gebiets weiterhin klar, das die Wahl der Ausgangsstoffe besonders vorteilhaft so erfolgt, dass mit dem Entfernen des Alkohols aus der Reaktionsmischung das Gleichgewicht auf die Seite der Produkte verschoben werden kann. Die Entfernung des Alkohols kann destillativ durch seinen im Vergleich zum verwendeten Amin niedrigeren Siedepunkt und/oder durch die Bildung eines Azeotrops erfolgen.

Als Polymerisationsinhibitoren kommen beispielsweise Hydrochinon, 4-Hydroxy-2,2,6,6-tetra-methylpiperidinooxyl oder auch Bis(2-methoxycarbonylpropyl)sulfid oder Hydrochinonmonomethylether in Verbindung mit Sauerstoff in Frage.

Das eingesetzte Amin kann Wasser enthalten. Die Menge an Wasser im eingesetzten Amin liegt im Falle von Amin zwischen 50 und 500 ppm (0,05 - 0,005 Gew.-%). Das Amin wird vor dem Eintreten in den Reaktionsapparat bevorzugt über die Destillationskolonne (2) destillativ entwässert. Dabei wird das im Amin enthaltene Wasser über Kopf abgezogen. Zur Vermeidung einer Verunreinigung des Niedersieder-Austrags (13) mit dem eingesetzten Amin erfolgt die Aufgabe des Amins bevorzugt im unteren Teil der Destillationskolonne (2). Das eingesetzte Amin kann auch auf andere Art und Weise entwässert werden:
- durch eine vorgeschaltete Entwässerungs-Destillationskolonne oder
- durch Behandlung mit einem Entwässerungsmittel, wie beispielsweise ein Molekularsieb,
   oder
- durch ein Membrantrennverfahren, wie beispielsweise eine Pervaporation.

Die Entwässerung ist daher bedeutsam, da das im Amin enthaltene Wasser zur irreversiblen Schädigung des Katalysators (z.B. Tetraalkyltitanat) im Reaktor führen kann. Das im Amin enthaltene Wasser führt zur Bildung von Nebenprodukten und ist daher strikt zu vermeiden. Durch diesen Entwässerungsschritt vermeidet man die Hydrolyse des Katalysators und die damit entstehenden Kosten durch erhöhte Katalysatoreinsatzmengen und durch Probleme mit Feststoffniederschlägen. Außerdem wird die Reinheit des Produktes durch einen verringerten Anteil an Nebenprodukten erhöht.

Die Umsetzung erfolgt im Reaktionsapparat (1) bei einer Temperatur im Bereich zwischen 80 und 180 °C je nach Stoffsystem und Betriebsdruck. Bevorzugt ist der Temperaturbereich zwischen 110 und 160 °C. Zur Erhöhung der Reaktionsgeschwindigkeit wird der bei der Reaktion frei werdende Alkohol über die Destillationskolonne (2) aus dem Reaktionsgemisch, gegebenenfalls auch als Azeotrop mit dem Alkohol abgezogen (13). Dies kann sowohl bei atmosphärischem Druck, bei Überdruck oder bei Unterdruck erfolgen. Die Reaktionsmischung, welche größtenteils aus dem Produkt-Alkylacrylatamid, nicht umgesetztem Acrylat und Amin sowie geringen Mengen Alkohol, dem Katalysator, den Polymerisationsinhibitoren und einem Anteil an Nebenprodukten besteht, wird nach ca. 0,5 - 3 Stunden Reaktorverweilzeit (bevorzugt ist eine Verweilzeit von 1 - 2 Stunden) einem kontinuierlich betriebenen Fallfilmverdampfer (5) zugeführt. Die Brüden des Fallfilmverdampfers (5) werden einer Niedrigsieder-Destillationskolonne (3) zugeführt. Dort erfolgt bei vermindertem Druck, bevorzugt im Bereich von ca. 1 - 500 mbar, die Abtrennung der in Bezug auf das Produktamid niedrigsiedenden Komponenten, vorwiegend Produktalkohol und nicht umgesetztes Edukt-Acrylat und -Amin. Diese werden über den Kopf der Destillationskolonne (3) abgezogen und in den Reaktorbereich oder in die Destillationskolonne (2) zurückgeführt (14). Durch diesen Kreislaufstrom wird ein hoher Umsatz bezogen auf die Edukte und den gesamten Prozess erzielt.

Das im Ablauf des Fallfilmverdampfers (5) anfallende mit Katalysator, Polymerisationsinhibitor und hochsiedenden Nebenprodukten noch verunreinigte Roh-Amid (15) enthält vorzugsweise > 80 Gew.-% Produktamid und wird zur Aufarbeitung einer weiteren Vakuumdestillationsstufe, welche im bevorzugten Druckbereich zwischen 0,1 und 200 mbar arbeitet, zugeführt. Hier erfolgt die destillative Abtrennung des hochreinen Produktamids als Kopfprodukt.

Die im Prozeß gebildeten Nebenprodukte stellen in Bezug auf das Eduktamin und das Edukt-Acrylat hochsiedende Komponenten dar und gelangen somit als Verunreinigung in das Produktamid, wodurch die Produktqualität deutlich gesenkt wird. Dieses Problem kann dadurch gelöst werden, dass man zur Abtrennung des Produktamids vom Katalysator und den Polymerisationsinhibitoren sowie den hochsiedenden Nebenprodukten einen Apparat mit schonender Filmverdampfung wie (5) einsetzt. Als geeignete Apparate sind hierfür Fallfilm-, Dünnschicht- und Kurzwegverdampfer bekannt.

An die Herstellung der Alkylaminoacrylamide kann sich gegebenenfalls noch eine Reindestillationsanlage anschließen, die auch unter vermindertem Druck, beispielsweise bei 500-0,1 mbar, betrieben werden kann.

Das erfindungsgemäße Verfahren wird durch nachfolgendes Beispiel näher erläutert, ohne darauf beschränkt zu sein.

### Beispiel: Kontinuierliche Aminolyse zu Aminoacrylamid

Zur kontinuierlichen Herstellung von N-Dimethylaminopropylacrylamid wurde dem 1. Reaktionskessel 200 kg/h Butylacrylat/Katalysator-Feed mit einem Anteil von 2,0 Gew.-% Isopropyl-Titanat 5,0 Gew.-% Dioctylzinnoxid der Destillationskolonne (2) und 144 kg/h N-Dimethylaminopropylamin (DMAPA) zudosiert. Weiterhin floss dem 1. Reaktionskessel über die Destillationskolonne (2) der Kreislaufrückstrom vom Kopf der Niedrigsieder-Destillationskolonne kontinuierlich zu (400 kg/h mit der Zusammensetzung 70 Gew.-% Eduktacrylat sowie Butylalkohol, DMAPA und Nebenprodukte. Das molare Butylacrylat : DMAPA-Verhältnis im Reaktorzulauf betrug 1,8 : 1. Weiterhin liefen die in der Destillationskolonne (2) vom Butanol befreiten Brüden der Rührkessel über den Kolonnensumpf dem 1. Reaktionskessel zu. Unter diesen Reaktionsbedingungen (Druck ca. 500 mbar) stellte sich eine Reaktionstemperatur von 138°C im 1. Reaktionskessel ein. Die Reaktionstemperatur im 2. und 3. Reaktionskessel betrug 143 bzw. 155 °C. Der Destillat-Abzug der Destillationskolonne (2) betrug 110 kg/h. Der Ablauf des 1. Reaktionskessels lief in den 2. Reaktionskessel und der Ablauf des 2. Reaktionskessels lief in den 3. Reaktionskessel. Bei einer Verweilzeit von insgesamt ca. 150 Min. wurden am Ablauf des 3. Reaktionskessels folgende Anteile der Komponenten bestimmt:

| | |
|---|---|
| Butylacrylat | 43 Gew.-% |
| DMAPA | 4,86 Gew.-% |
| Aminoamid | 35 Gew.-% |

Die Brüden der einzelnen Reaktionskessel wurden kontinuierlich der Destillationskolonne (2) zugeführt.

Der Ablauf des 3. Reaktionskessels lief kontinuierlich dem Dünnschichtverdampfer einer Niedrigsieder-Kolonne zu, in der nicht umgesetztes DMAPA, Butylacrylat und Butanol als Destillat (400 kg/h) abgezogen und als Kreislaufrückstrom der Destillationskolonne (2) wieder zugeführt wurden. Der Sumpf-Auslauf des Dünnschichtverdampfers der Niedrigsiederkolonne betrug 240 kg/h und hatte die Zusammensetzung: ca. 90 % Produktamid, 0,1 % DMAPA, einen grösseren Anteil hochsiedender Komponenten und Spuren der Reaktanden.

## Patentansprüche

1. Verfahren zur kontinuierlich betriebenen Herstellung von Alkylaminoacrylamid der Formel (C), 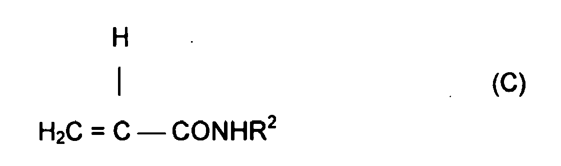 worin R² für einen linearen oder verzweigten oder cyclischen Alkylrest oder Arylrest mit 1 bis 12 C-Atomen steht, durch Umsetzung einer Verbindung der Formel (B),
R²NH₂ (B)
wobei R² die oben angegebene Bedeutung besitzt, mit Alkylacrylat (A), 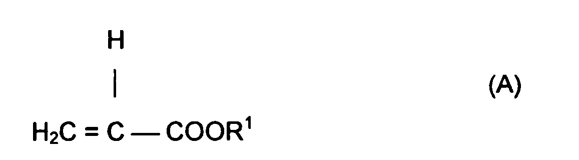 worin R¹ für einen linearen oder verzweigten Alkylrest mit 3 bis 10 Kohlenstoffatomen steht, in Gegenwart eines Katalysators oder eines Katalysatorgemischs und in Gegenwart mindestens eines Polymerisationsinhibitors in einer Apparatur zur kontinuierlichen Umsetzung,
**dadurch gekennzeichnet,**
**dass** die Reaktanden einem geeigneten Reaktionsapparat (1) kontinuierlich zugeführt werden und dass der bei der Reaktion entstehenden Alkohol bzw. ein Alkohol/Akylacrylat-Gemisch (13) kontinuierlich mit Hilfe einer Destillationskolonne (2) abgezogen wird und außerdem:
- das Reaktionsgemisch kontinuierlich aus dem Reaktionsapparat in eine Destillationskolonne (3) bzw. einen Verdampfer (5) geführt wird, wobei durch Destillation unter vermindertem Druck über Kopf die leicht flüchtigen Komponenten (A, B, Alkohol) und ein sehr geringer Anteil Produktamid (C) abgezogen und in den Reaktionsapparat zurückgeführt werden und aus dem Sumpf der Kolonne die Produktamide (C) zusammen mit dem Katalysator und den Polymerisationsinhibitoren sowie hochsiedenden Nebenprodukten abgezogen werden;
- der Sumpfstrom (15) aus der Destillationskolonne (3) einer Reindestillation kontinuierlich zugeführt wird,
- der Katalysator oder das Katalysatorgemisch in Mengen von 0,1-10 Gew.%, bezogen auf das eingesetzte Acrylat, eingesetzt wird,
- die Umsetzung im Reaktionsapparat (1) bei einer Temperatur im Bereich zwischen 80 und 180 °C erfolgt,
- und die Verweilzeit im Reaktionsapparat zwischen 0,5 und 3 Stunden beträgt.

2. Verfahren nach Anspruch 1,
**dadurch gekennzeichnet,**
**dass** der Brüdenstrom des Verdampfers (5) einer weiteren Destillationskolonne kontinuierlich zugeführt wird, wobei durch Destillation unter vermindertem Druck über Kopf das hochreine Produktamid (C) abgeführt wird und über den Sumpf der Katalysator und die Polymerisationsinhibitoren sowie die hochsiedenden Nebenprodukte mit einem kleinen Teil Produktamid (C) abgezogen werden.

3. Verfahren nach einem der vorstehenden Ansprüche,
**dadurch gekennzeichnet,**
**dass** das Amin (B) zur Entwässerung über die Destillationskolonne (2) dem Reaktionsapparat zugeführt wird.

4. Verfahren nach einem der vorstehenden Ansprüche,
**dadurch gekennzeichnet,**
**dass** das molare Verhältnis von Alkylacrylat zu Amin im Zulauf zwischen 1 und 2, vorzugsweise 1,05-1,15 beträgt.

5. Verfahren nach einem der vorstehenden Ansprüche,
**dadurch gekennzeichnet,**
**dass** man als Katalysator ein Tetraalkyltitanat einsetzt.

6. Verfahren nach einem der Ansprüche 1 bis 5,
**dadurch gekennzeichnet,**
**dass** der Katalysator in einer Menge von 0,2 - 7 Gew.-%, bezogen auf eingesetztes Acrylat eingesetzt wird.

7. Verfahren nach einem der vorstehenden Ansprüche,
**dadurch gekennzeichnet,**
**dass** man als Katalysatormischung eine Mischung aus Dioctylzinnoxid und Isopropyltitanat im Verhältnis 2,5 : 1 (Gew.-% /Gew.-%) anwendet.

8. Verfahren nach einem der vorstehenden Ansprüche,
**dadurch gekennzeichnet,**
**dass** man als Polymerisationsinhibitor entweder Phenothiazin, Tertiärbutylcatechol, Hydrochinonmonomethylether, Hydrochinon oder deren Gemische einsetzt, wobei die Menge des Inhibitor zwischen 100 und 5000 ppm, bezogen auf das Reaktionsgemisch, beträgt.

9. Verfahren nach einem der vorstehenden Ansprüche,
**dadurch gekennzeichnet,**
**dass** man als Polymerisationsinhibitor zusätzlich noch Sauerstoff verwendet.

10. Verfahren nach einem der vorstehenden Ansprüche,
**dadurch gekennzeichnet,**
**dass** man als Amin vorzugsweise Dimethylaminopropylamin verwendet.

11. Verfahren nach einem der vorstehenden Ansprüche,
**dadurch gekennzeichnet,**
**dass** der Druck in der ersten Destillationskolonne (3) zwischen 2 und 500 mbar beträgt.

12. Verfahren einem der vorstehenden Ansprüche,
**dadurch gekennzeichnet,**
**dass** der Verdampfer (5) ein Filmverdampfer ist.

## Claims

1. Process for continuously preparing alkylamino acrylamide of the formula (C) 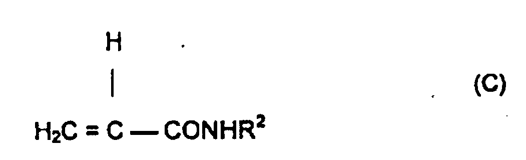 where R² is a linear or branched or cyclic alkyl radical or aryl radical having from 1 to 12 carbon atoms by reacting a compound of the formula (B)
R²NH₂ (B)
where R² is as defined above with alkyl acrylate (A) 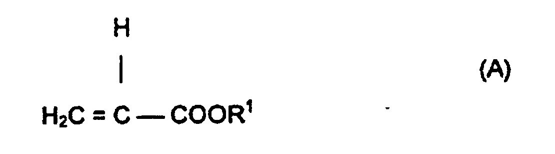 where R¹ is a linear or branched alkyl radical having from 3 to 10 carbon atoms, in the presence of a catalyst or a catalyst mixture and in the presence of at least one polymerization inhibitor in an apparatus for continuous transesterification,
**characterized in that**
the reactants are continuously fed to a suitable reaction apparatus (1) and that the alcohol or an alcohol/alkyl acrylate mixture (13) resulting from the reaction is continuously removed with the aid of a distillation column (2), and also:
- the reaction mixture is continuously conducted from the reaction apparatus into a distillation column (3) or an evaporator (5) in which distillation under reduced pressure is used to remove the volatile components (A, B, alcohol) and a very low proportion of product amide (C) overhead which are recycled into the reaction apparatus and to remove the product amides (C) together with the catalyst and the polymerization inhibitors and also high-boiling by-products from the bottom of the column;
- the bottom stream (15) from the distillation column (3) is fed continuously to a purifying distillation,
- the catalyst or the catalyst mixture is used in amounts of 0.1-10% by weight, based on the acrylate used,
- the reaction in reaction apparatus (1) is effected at a temperature in the range between 80 and 180°C,
- and the residence time in the reaction apparatus is 0.5 to 3 hours.

2. Process according to Claim 1,
**characterized in that**
the vapour stream of the evaporator (5) is fed continuously to a further distillation column in which distillation under reduced pressure is used to remove the highly pure product amide (C) overhead and to remove the catalyst and the polymerization inhibitors and also the high-boiling by-products with a small portion of product amide (C) via the bottom.

3. Process according to either of the preceding claims,
**characterized in that**
the amine (B) is fed to the reaction apparatus via the distillation column (2) for dewatering.

4. Process according to any of the preceding claims,
**characterized in that**
the molar ratio of alkyl acrylate to amine in the feed is from 1 to 2, preferably 1.05 - 1.15.

5. Process according to any of the preceding claims,
**characterized in that**
the catalyst used is a tetraalkyl titanate.

6. Process according to any of Claims 1 to 5,
**characterized in that**
the catalyst is used in an amount of 0.2-7% by weight, based on acrylate used.

7. Process according to any of the preceding claims,
**characterized in that**
the catalyst mixture employed is a mixture of dioctyltin oxide and isopropyl titanate in a ratio of 2.5:1 (% by wt./% by wt.).

8. Process according to any of the preceding claims,
**characterized in that**
the polymerization inhibitor used is either phenothiazine, tert-butylcatechol, hydroquinone monomethyl ether, hydroquinone or a mixture thereof in an amount of 100 to 5000 ppm, based on the reaction mixture.

9. Process according to any of the preceding claims,
**characterized in that**
oxygen is additionally used as a polymerization inhibitor.

10. Process according to any of the preceding claims,
**characterized in that**
the amine used is preferably dimethylaminopropylamine.

11. Process according to any of the preceding claims,
**characterized in that**
the pressure in the first distillation column (3) is 2 to 500 mbar.

12. Process according to any of the preceding claims,
**characterized in that**
the evaporator (5) is a film evaporator.

## Revendications

1. Procédé pour la fabrication en continu d'alkylaminoacrylamide de formule (C) : 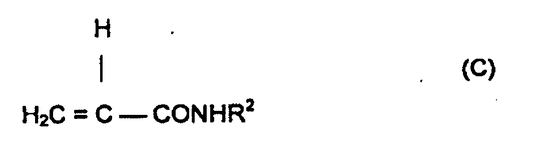 dans laquelle R² représente un radical alkyle ou aryle linéaire ou ramifié ou cyclique, ayant 1 à 12 atomes de carbone, consistant à faire réagir un composé de formule (B) :
R²NH₂ (B)
dans laquelle R² a la signification indiquée ci-dessus, avec d'acrylate d'alkyle de formule (A) : 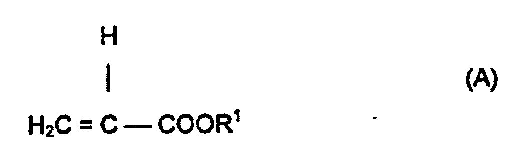 dans laquelle R¹ représente un radical alkyle linéaire ou ramifié, ayant 3 à 10 atomes de carbone, en présence d'un catalyseur ou d'un mélange de catalyseurs et en présence d'au moins un inhibiteur de polymérisation dans un appareil permettant d'effectuer la transestérification en continu,
**caractérisé en ce que** :
les réactifs sont acheminés en continu à un appareil réactionnel approprié (1) et **en ce que** l'alcool ou un mélange d'alcool/acrylate d'alkyle (13) formé lors de la réaction est extrait de manière continue, à l'aide d'une colonne de distillation (2) et se **caractérise par** ailleurs en ce que :
- le mélange réactionnel est acheminé de manière continue de l'appareil réactionnel vers une colonne de distillation (3) ou un évaporateur (5), opération pendant laquelle, en réalisant une distillation sous pression réduite, les composants aisément volatils (A, B, alcool) et une très faible fraction de l'amide produit (C) sont extraits en tête de colonne et recyclés dans l'appareil réactionnel, et les amides produits (C), conjointement avec le catalyseur et les inhibiteurs de polymérisation, ainsi que les produits secondaires à point d'ébullition élevé, sont extraits en bas de colonne ;
- le courant de bas de colonne (15) provenant de la colonne de distillation (3) est amené en continu à une distillation purificatrice,
- le catalyseur ou le mélange de catalyseurs est utilisé en quantités de 0,1 à 10 % en poids, par rapport à l'acrylate utilisé,
- la réaction dans l'appareil de réaction (1) est effectué à une température dans la plage comprise entre 80 et 180 °C,
- et le temps de séjour dans l'appareil réactionnel est compris entre 0,5 et 3 heures.

2. Procédé selon la revendication 1,
**caractérisé en ce que** :
le courant de fumées de l'évaporateur (5) est acheminé en continu à encore une colonne de distillation, opération pendant laquelle l'amide produit (C) de pureté élevée est évacué par distillation sous pression réduite en tête de colonne, tandis que le catalyseur et les inhibiteurs de polymérisation, ainsi que les produits secondaires à point d'ébullition élevé sont extraits, avec une petite partie de l'amide produit (C), en bas de colonne.

3. Procédé selon l'une des revendications précédentes,
**caractérisé en ce que** :
l'amine (B) est acheminée à l'appareil réactionnel pour l'élimination de l'eau via la colonne de distillation (2).

4. Procédé selon l'une des revendications précédentes,
**caractérisé en ce que** :
le rapport molaire d'acrylate d'alkyle à l'amine dans l'alimentation est compris entre 1 et 2, de préférence entre 1,05 et 1,15.

5. Procédé selon l'une des revendications précédentes,
**caractérisé en ce que** :
on utilise un titanate de tétraalkyle comme catalyseur.

6. Procédé selon l'une des revendications 1 à 5,
**caractérisé en ce que** :
le catalyseur est utilisé en quantité de 0,2 à 7 % en poids par rapport à l'acrylate utilisé.

7. Procédé selon l'une des revendications précédentes,
**caractérisé en ce que** :
un mélange d'oxyde de dioctyle étain et de titanate d'isopropyle est utilisé en tant que mélange de catalyseurs en un rapport de 2,5:1 (% en poids/% en poids).

8. Procédé selon l'une des revendications précédentes,
**caractérisé en ce que** :
on utilise comme inhibiteur de polymérisation, la phénothiazine, le butylcatéchol tertiaire, le monométhyléther d'hydroquinone, l'hydroquinone ou leurs mélanges, la quantité de l'inhibiteur étant comprise entre 100 et 5000 ppm par rapport au mélange réactionnel.

9. Procédé selon l'une des revendications précédentes,
**caractérisé en ce que** :
on utilise encore de l'oxygène supplémentaire comme inhibiteur de polymérisation.

10. Procédé selon l'une des revendications précédentes,
**caractérisé en ce que** :
on utilise, de préférence, la diméthylaminopropylamine comme amine.

11. Procédé selon l'une des revendications précédentes,
**caractérisé en ce que** :
la pression dans la première colonne de distillation (3) est comprise entre 2 et 500 mbars.

12. Procédé selon l'une des revendications précédentes,
**caractérisé en ce que** :
l'évaporateur (5) est un évaporateur à film.
